# EUROPEAN PATENT SPECIFICATION

(11) **EP 0 997 107 B1**
(45) Date of publication and mention of the grant of the patent: **22.12.2004**
(21) Application number: 99306222.3
(22) Date of filing: 05.08.1999
(51) Int. Cl.: A61B 17/68

(54) **Orthopedic lock nut**
Orthopädische selbsthemmende Mutter
Ecrou auto-freiné orthopédique

(30) Priority: 06.08.1998 US 130268
(43) Date of publication of application: 03.05.2000
(73) Proprietor: DePuy ACE Medical Company, El Segundo, CA 90245 (US)
(72) Inventor: Hayes, S. Kyle, Mission Viejo California 92691 (US)
(74) Representative: Fisher, Adrian John

(56) References cited:
- EP-A- 0 685 206
- FR-A- 2 729 074
- US-A- 5 108 399
- US-A- 5 409 486
- DATABASE WPI Week 199813 Derwent Publications Ltd., London, GB; AN 1998-138572 XP002129118 & JP 10 014936 A (HOMUZU GIKEN)

## Description

### BACKGROUND OF THE INVENTION

The present invention relates to nuts for screws, and more particularly, for orthopedic nuts to be used to better secure a bone screw such as through osteoporotic bone.

Bone screws are used in many applications in orthopedic surgery. For instance, bone screws may be used to secure a strengthening or support member such as a bone plate to a fractured bone. Bone screws may be used to secure intramedullary nails into the intramedullary cavity. The bone screws include a head which can be rotated or torqued, and a shaft with threads. Some bone screws include a self-tapping portion which cuts threads in the bone upon rotational driving of the bone screw into the bone.

Bone screws may exert a force parallel to the longitudinal axis of the screw, or a force transverse to the longitudinal axis of the screw. Bone screws may be used to attach two portions of a fractured bone together, and may have thread forms particularly intended to exert a longitudinal force and pull two longitudinally separated portions together. When used with an "active compression plate", the screw hole in the plate may place a transverse force on a bone screw which is transferred through the bone screw to the bone.

The ability of a screw to hold in bone, i.e., the "pullout" strength of a screw, is dependent on the strength of the screw threads, the major diameter, minor diameter and the pitch of the screw threads, and the shear strength of the bone that the screw is being placed into. The shear strength of bone is dependent upon the type, density and health of the bone. Cancellous bone is generally less dense than cortical bone and therefore has less shear strength. Bone screws intended for cancellous bone commonly have different screw threads than bone screws intended for cortical bone. To get more bone between the threads for a greater pullout strength when the bone is weaker, thread forms designed for cancellous bones generally have a greater pitch and a greater difference between major and minor diameters. In some patients the bone density or bone stock is poor or osteoporotic and has a lower shear strength than healthy bone. Cortical bone that is osteoporotic is weaker than normal healthy cortical bone, but may still be stronger than healthy cancellous bone.

In low shear strength bone, both conical bone screws and cancellous bone screw can "back off" or become loose in the bone over time. The backing off of a bone screw can have numerous deleterious effects. The bone plate, intramedullary nail or other strengthening member attached by the bone screw may migrate from its intended location. The bone screw may "toggle" or move relative to the bone in which it is attached, which greatly increases the stress concentration at the threads. This can lead to premature fatigue failure either of the screw threads or of mating threads in the bone. Toggling may also cause a portion of the bone screw which is intended to be placed in double shear to only transmit a single shear force. Particularly with cancellous bone screws with a small minor diameter, placing the screw in single shear can lead to premature fatigue failure of the screw shaft.

In some situations, the bone screw extends fully through the bone, such that the threaded portion projects out the opposite side of the bone. In these situations, the bone screw can be further secured to the bone with an orthopedic nut. The orthopedic nut distributes loading on the underside of the nut over an area of the bone instead of relying solely on the pullout strength of the screw in the bone. Thus, orthopedic nuts give surgeons another option to secure a screw in weaker bones and still allow the surgeon to opt for either cortical screws or cancellous screws. Current orthopedic nuts are formed from stainless steel with a thread form which is oversized so it will easily turn up the screw.

In FR-A-2 729 074, there is disclosed an orthopedic nut of the type specified in the preamble of the accompanying claim 1.

In EP-A-0 685 206, there is disclosed an orthopedic nut with a plastics insert to provide a self-locking capability.

### BRIEF SUMMARY OF THE INVENTION

In accordance with the present invention there is provided is an orthopedic nut for a bone screw as set forth in the accompanying claim 1. The polymer may be compressible relative to the materials typically used in bone screws, and thus forms a locking function on the bone screw.

Further aspects of the invention are set out in the accompanying claims 2 to 9.

### BRIEF DESCRIPTION OF THE DRAWINGS

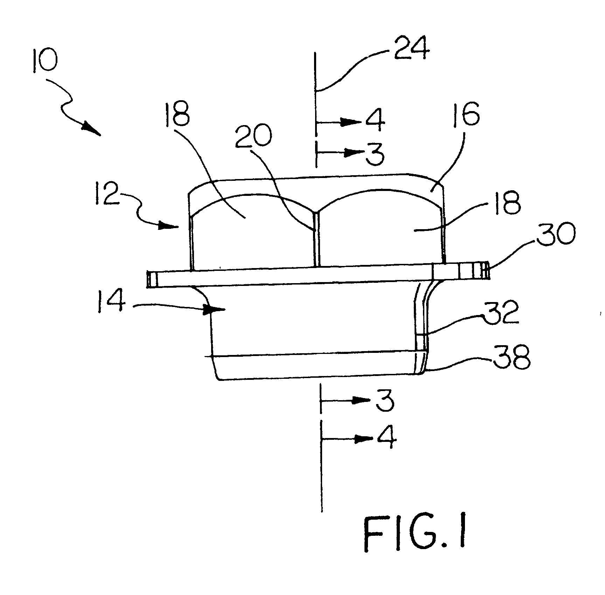

FIG. 1 is a side view of an orthopedic nut of the present invention.

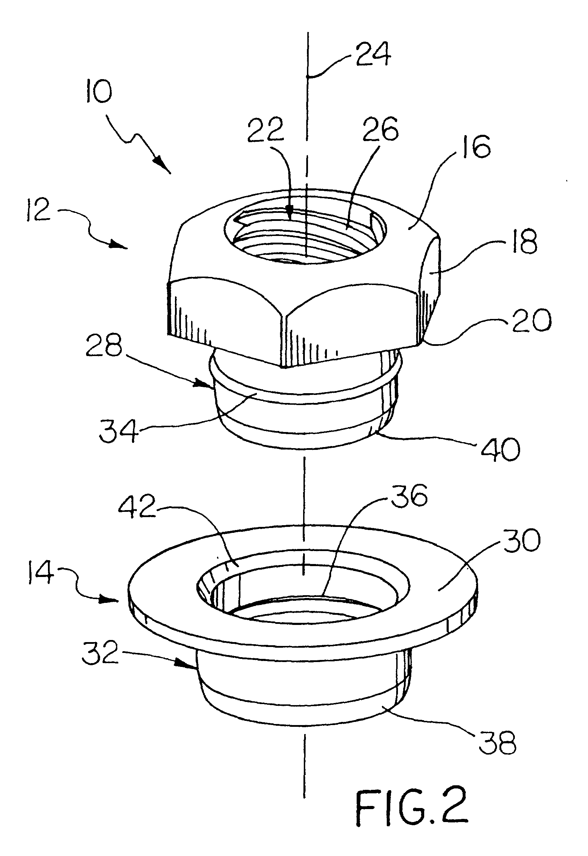

FIG. 2 is an explosive perspective view of the orthopedic nut of FIG. 1.
FIG. 3 is a cross-sectional view of the head portion taken along lines 3-3 in FIG. 1.
FIG. 4 is a cross-sectional view of the washer portion taken along lines 4-4 from FIG. 1.
FIG. 5 is a cross-sectional view of a second alternative design of the head portion.
FIG. 6 is a cross-sectional view of a third alternative design of the head portion.
FIG. 7 is a plan view of a fourth alternative embodiment of the head portion having a polymer pin insert.
FIG. 8 is an exploded perspective view of the embodiment of FIG. 7.
FIG. 9 is an exploded perspective view of a fifth alternative embodiment of the head portion.

While the above-identified drawing figures set forth preferred embodiments, other embodiments of the present invention are also contemplated, some of which are noted in the discussion. In all cases, this disclosure presents the illustrated embodiments of the present invention by way of representation and not limitation. Numerous other minor modifications and embodiments can be devised by those skilled in the art which fall within the scope of this invention as defined by the accompanying claims.

### DETAILED DESCRIPTION

The orthopedic nut 10 of the present invention includes a head portion 12 and a washer 14. The nut 10 is intended to be used with an orthopedic bone screw (not shown), which may be formed of a metal such as stainless steel or titanium Because of the desired high strength of the bone screw shaft and the bone screw threads, the bone screw material generally has a low compressibility.

The head portion 12 is formed of a polymer which is softer and has a higher compressibility than typical bone screws. For instance, the polymer material may have a hardness on the Shore A, B or D scales, which the material of the bone screws may have a hardness on the Rockwell C scale, such as in the range of 30-35 Rockwell C. The relatively high compressibility of the polymer material allows the nut 10 to exert a compression spring force against the bone screw when the nut 10 is tightened relative to the bone screw, and the compression spring force resists backing off under vibration.

The polymer material of the head portion 12 should be bio-compatible over a long period of time, such as up to a year or more. For instance, the head portion 12 may be formed of polymer materials which have been approved or previously used in the body, such as polyethylene, acetal copolymer (DELRIN), polysulfone (LEXAN), polytetrafluoroethylene (PTFE), propylene, certain polyesters, cellulose acetate, polybutyl diene, polycarbonate, polybutester, GORTEX, etc.

The preferred polymer material is an ultra high molecular weight polyethylene known as HYLEMER developed by DePuy/DuPont. The ultra high molecular weight polyethylene is easily formed as an extrusion to ASTM - 648. After forming, the ultra high molecular weight polyethylene can be machined with single, triple or double lead threads. In comparison to other polymers, the ultra high molecular weight polyethylene is a strong, hard material, such as a hardness of the range of Shore 60-70. At the same time, the ultra high molecular weight polyethylene is soft and compressible relative to the titanium or stainless steel materials of typical bone screws.

The head portion 12 preferably has a head 16 which is designed to be received in a torqueing tool (not shown). The head 16 should be large enough to provide strength to transmit the necessary torque to the head portion 12, but not so large as to permit interference between the torqueing tool and orthopedic nuts from neighboring bone screws. In the preferred embodiment, the head portion 12 has a hexagonal head 16 with about a 0.39 inch (9.9mm) diameter across flats 18 and about a 0.45 inch (11.4mm) diameter across corners 20, so as to be received with a slight clearance in a standard 10mm wrench (not shown) from the ACE FISHER external fixation system. Workers skilled in the art will appreciate that the head portion 12 may be shaped in a wide variety of other ways to as to be receivable in a correspondingly shaped torqueing tool.

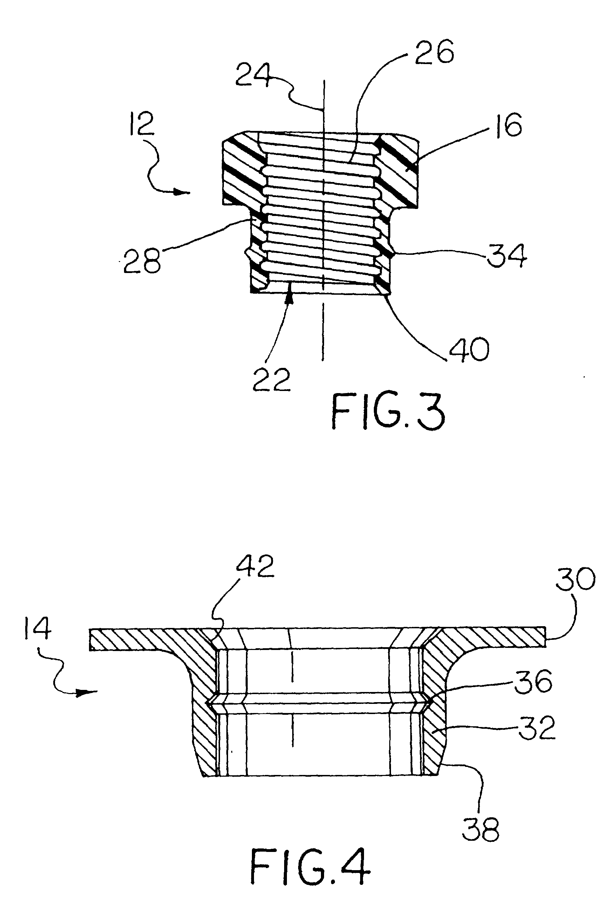

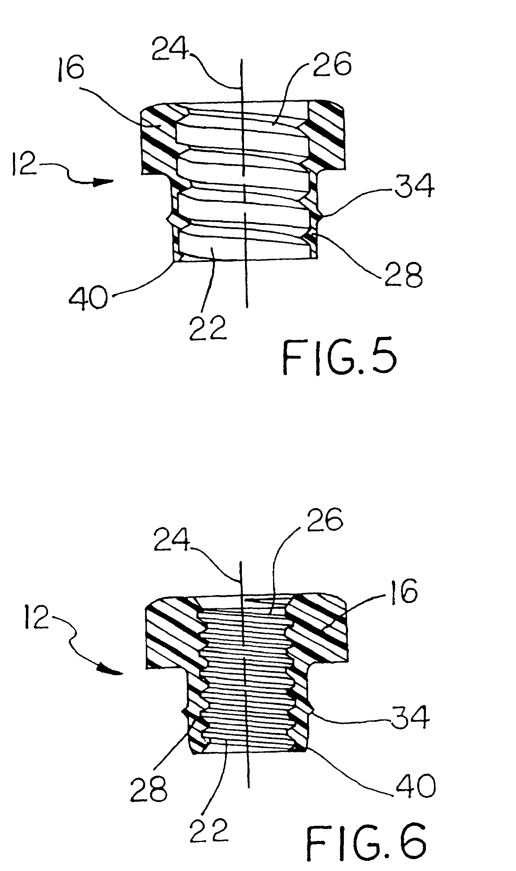

The preferred head portion 12 includes a screw hole 22 defined therethrough about a longitudinal head axis 24. The head portion 12 has threads 26 to mate with a selected bone screw, such as a 4.5 mm cortical screw, a 6.5 mm cortical screw or a 6.5 mm cancellous screw, and thus a variety of different thread designs may be used. FIG. 3 shows a preferred triple lead thread as may be correspond to a 6.5 mm cortical bone screw. The multiple lead thread 26 allows the bone screw and the nut 10 to advance upon rotation toward each other at a high rate, while providing large contact area between the threads of the bone screw and the threads 26 of the nut 10. FIG. 5 shows a larger single lead thread form which will appropriately mate with a thread on a 6.5mm cancellous bone screw. FIG. 6 shows a third embodiment with a double lead thread.

The threads 26 may be molded or machined into the head portion 12 during manufacture of the head portion 12. Alternatively, particularly because the polymer material of the head portion 12 is softer than the material of typical bone screws, the threads 26 may be cut into the head portion 12 by the bone screw itself. In many instances, the polymer material of the head portion 12 will be softer than healthy bone material, and self-tapping of the bone screw through the head portion 12 will not significantly increase the torque necessary to drive the bone screw through the bone and head portion 12. The head portion 12 as manufactured may have a smooth cylindrical screw hole 22 with an inside diameter which matches the minor diameter of the intended bone screw. The head portion 12 may alternatively have the beginnings of thread forms, which are still smaller than the major diameter of the intended bone screw. As a third alternative, the head portion 12 may be manufactured without a screw hole 22 at all, with the entirety of the screw hole 22 cut by the self-tapping bone screw. As long as the self-tapping bone screw cuts at least a portion of the threads 26, there will be a tight attachment between the head portion 12 and the bone screw to prevent any toggling or vibration of the bone screw relative to the head portion 12.

The nut 10 is intended to be used with a bone screw with a standard thread form built to ISO specifications. With such a standard thread form, the bone screw will be radiused at its major and minor diameters. In contrast, the head portion 12 may have sharp edges cut at the major and/or minor diameter of threads 26 to provide a slight interference fit. Alternatively or in conjunction with the difference in thread form shapes, the major and/or minor diameters of the screw hole 22 may be slightly smaller than the corresponding major and/or minor diameters of the bone screw to provide a slight interference fit. For example, the preferred embodiment has threads 26 cut with a minor diameter of about 0.15 inches (3.8mm) and a major diameter of about 0.18 inches (4.6mm). When mated with a standard bone screw, the screw hole 22 of the preferred nut 10 has a slight clearance on the minor diameter, but a slight interference at the major diameter. The preferred major diameter interference is 0.002 to 0.008 inches (0.05 to 0.20mm), such as a nominal 0.005 inch interference with a 0.003 inch major diameter tolerance (0.13 ± 0.08mm). Such an interference at major diameter/clearance at minor diameter configuration insures that the bone screw will cut at least a portion of the threads 26 into the head portion 12 for a secure, non-toggling fit, but the polymer chips formed by such cutting will be caught within the minor diameter clearance and not released into the patient's body.

The nut 10 preferably has an overall low profile design, so as to provide minimal agitation to the tissue on the opposite side or nut side of the bone. To provide a low profile, the head portion 12 includes a sleeve 28 which extends longitudinally from the head 16. The sleeve 28 extends within the washer 14 and within the screw hole in the bone, so a long threaded length is provided without a high profile on the nut 10. For instance, the sleeve 28 may permit the thread length to be two or more times the length of the head 16. In a preferred embodiment, the head portion 12 is about 0.31 inches (7.9mm) long, which is made up of a 0.14 inch (3.6mm) long head 16 with a 0.17 inch (4.3mm) long sleeve 28. The long thread length allows the head portion 12 to have a very high pullout strength even when using a lower strength compressible polymer material for the head portion 12.

The wall thickness of the head sleeve 28 is preferably as thin as possible while still maintaining appropriate longitudinal strength for the threads 26. A preferred wall thickness of the head sleeve 28 is on the order of .03 inches (0.7mm) larger than the major diameter. That is, for a head portion 12 with threads 26 having a major diameter of 0.18 inches (4.6mm), the head sleeve 28 may have an outer diameter of about 0.23 inches (5.9mm).

The washer 14 includes a shoulder 30 and, preferably, a washer sleeve 32. The outer diameter of the shoulder 30 is designed to be as large as possible, as a larger washer diameter will give the larger surface area for bearing contact with the underlying bone. A large contact area helps distribute the loading under the washer 14 against the bone and aids in fixation against osteoporotic bone. However, the shoulder 30 should not be so large to interfere with side by side use with an adjacent bone screw. For instance, the distal locking hole spacing of a typical intramedullary nail may be 0.79 inches (20 mm) between holes. For use between such holes, the shoulder outer diameter needs to be 0.79 inches (20mm) or less. The preferred outer diameter is about 0.52 inches (13.2mm). This outer diameter provides a larger contact area than would be possible with merely the head portion 12, because the shoulder 30 of the washer 14 does not need to be received in a torqueing tool and need not provide clearance for the torqueing tool.

The preferred shoulder 30 is flat and planar, so as to mate as best possible to a wide variety of underlying bone shapes. If desired, the shoulder 30 may alternatively be contoured, to better mate and bear against bone having a corresponding contour. The preferred shoulder 30 has a circular outer shape, but ovular, square, hexagonal or other shapes may alternatively be used. If desired, the bottom surface of the shoulder may have a different contour and/or shape than the top surface of the shoulder, but forming the shoulder of a uniform thickness is preferred for ease of manufacturing.

The washer sleeve 32 surrounds and lends strength to the head sleeve 28. Accordingly, the inner diameter on the washer sleeve 32 corresponds to the outer diameter of the head sleeve 28 with a slight clearance. The wall thickness of the washer sleeve 32 is selected to provide appropriate strength. The washer 14 may be marked to show the manufacturer product number on the washer 14 and on the nut 10.

The washer 14 is formed of a bio-compatible materiaL Ultra high molecular weight polyethylene and most other polymer materials are radiolucent, and the washer 14 is preferably formed of a radiopaque material to aid in locating the orthopedic nut 10 and the back side of the bone when viewed on a fluoroscope or other viewing instrument. The washer material does not need a relatively high compressibility. While strength of the washer 14 is important, strength is not as significant of the washer material as for the bone screw. The preferred washer 14 is formed of titanium, such as TI-6Al-4V eli. This titanium which is bio-compatible and radiopaque, and provides ample strength. Using titanium, a preferred material thickness for the shoulder 30 is about 0.025 inches (0.64mm), and a preferred wall thickness for the washer sleeve 32 is also about 0.025 inches (0.64mm), which provides sufficient strength for the washer 14. Alternatively, washer 14 may be formed of other titaniums, such as commercially pure titaniums of grade 1 through 4, or of stainless steels, such as 316L, 316, or 22Cr-13Ni-5Mn stainless steeL Workers skilled in the art will appreciate that the washer 14 may alternatively be formed of a wide range of materials, with thicknesses appropriately selected for the strength of material used.

The washer 14 is preferably attached or attachable to but separately formed from the head portion 12. Attaching the washer 14 to the head portion 12 allows the surgeon to simultaneously install both the washer 14 and the head portion 12 onto a bone screw. Forming the washer 14 separate from the head portion 12 allows rotation of the washer 14 relative to the head portion 12. Because the washer 14 does not need to rotate with the head portion 12, rotation of the head portion 12 to tighten the nut 10 will not positively torque the washer 14. Instead, any rotation of the washer 14 is due to opposing frictional contact of the washer 14 with the head portion 12 as compared to the bone. With the head portion 12 formed of a ultra high molecular weight polyethylene and the washer 14 formed of titanium, and with planar surface interaction between the head 16 and the shoulder 30, a relatively low coefficient of friction is established between the head portion 12 and the washer 14. A high coefficient of friction will be present between the washer 14 and the coarse surface of the bone. Accordingly, the washer 14 will not further rotate with the head portion 12 after the washer 14 makes contact with the bone, and the washer 14 will generally not cause any grinding or wear damage to the underlying bone upon installation. Workers skilled in the art will appreciate that many alternative designs are available to allow the head portion 12 to easily rotate relative to the washer 14 depending on the materials and contours chosen, such as adding a bio-compatible lubricant between the head portion 12 and the washer 14. However, with the preferred configuration, no lubricant is necessary.

To rotationally attach the head portion 12 and the washer 14, the sleeve 28 of the head portion 12 may include a circumferential protrusion 34 sized and positioned to mate with a corresponding recess 36 in the washer sleeve 32. Due to the compressibility of the head portion material, the protrusion 34 may be simply deflected or depressed inward by the washer 14 to circumference of the head sleeve 28 during assembly of the washer 14 to the head portion 12. When the washer 14 is longitudinally pressed onto the head portion 12 a sufficient distance, the protrusion 34 snaps outward into the recess 36. The circumferential protrusion 34 received in the recess 36 allows free rotation of the washer 14 relative to the head portion 12, but prevents longitudinal movement of the washer 14 relative to the head portion 12. In the preferred embodiment, the protrusion 34 and corresponding recess 36 are a right triangle in cross section, extending about 0.01 inches (0.3mm) into the washer sleeve 32, with surfaces at 45° relative to the longitudinal axis 24.

Workers skilled in the art will appreciate that any number of alternative arrangements could be used to rotationally attach the washer 14 to the head portion 12. For instance, a plurality of longitudinally spaced circumferential protrusions could be used on the head sleeve 28 with a corresponding plurality of longitudinally spaced circumferential recesses on the washer sleeve 32. Such an attachment at a plurality of longitudinally spaced locations helps the wall of the head sleeve 28 to resist longitudinal creep under the stress of the bone screw. As further alternatives, the protrusion need not encircle the sleeve 28, or the protrusion may be positioned on the washer 14 rather than the head portion 12.

Alternatively, the washer 14 could include threads (not shown) to support each of the threads 26 on the head sleeve 28, such that the washer threads support a significant portion of the load from the bone screw. While such an arrangement provides thread strength advantages, the washer 14 would not be free to rotate relative to the head portion 12 and bone screw.

The sleeve 32 of the washer 14 may have an angled leading corner 38 along its outer diameter. In the preferred embodiment, the leading corner 38 of the washer 14 has an outer surface at a 75° cone angle to the longitudinal axis 24. The angled leading corner 38 aids in insertion of the nut 10 into the screw hole of the bone.

The head sleeve 28 may have an angled leading corner 40 along its outer diameter. The washer 14 may have an angled trailing corner 42 along its inner diameter. The angled leading corner 40 of the head sleeve 28 and the angled trailing corner 42 of the washer 14 aid in insertion of the head portion 12 into the washer 14 portion during assembly.

Assembly of the washer 14 to the head portion 12 may be done by the surgeon prior to attachment to the bone screw. Alternatively, the washer 14 may be placed around the bone screw prior to attachment of the head portion 12 to the bone screw, with attachment of the head portion 12 to the washer 14 achieved by advancing the head portion 12 along the bone screw. These methods of assembly are particularly beneficial for use with washers with shoulders of a particular shape for the area of attachment. For instance, the surgeon may be provided with washers having shoulders of a number of different shapes, to select the most appropriate shoulder shape for the site of attachment. If the washer is the preferred washer 14 having a circular, flat shoulder 30, the preferred method of assembly of the washer 14 to the head portion 12 is as a final step in manufacture, so it will not be necessary for the surgeon to separately perform this assembly step.

In the preferred method of use, the surgeon will fully thread the bone screw through the bone prior to positioning the nut 10. The lock nut 10 will then be threaded onto the stationary bone screw such as with a 10mm wrench. Alternatively, the washer 14 may be held in place prior to turning the bone screw through the bone, or the bone screw and the nut 10 may be simultaneously rotated in opposite directions.

The nut 10 is tightened onto the bone screw, with the sleeves 28, 32 advancing into the bone screw hole until the shoulder 30 makes bearing contact against the surface of the bone. Depending on the mating design between the threads of the bone screw and the threads 26 (if any) as manufactured on the head portion 12, tightening of the nut 10 onto a self-tapping bone screw may simultaneously cut threads 26 into the head portion 12. Once the shoulder 30 makes bearing contact against the surface of the bone, the nut may be further torqued relative to the bone screw an appropriate amount to place a spring compression force on the threads 26 of the head portion 12. The compressibility of the material of the head portion 12 allows the head portion 12 to lock the bone screw into place, and the bone screw will not back off such as under vibration.

Neither ultra high molecular weight polyethylene nor titanium biodegrade, and a surgeon may desire to remove the orthopedic nut 10 at the same time that the bone screw is removed, which may be a year or more after implantation. Alternatively, either or both of the head portion 12 and the washer 14 may be formed of a biodegradable polymer material. Exemplary biodegradable polymer materials include polylactic acids, poly-L-lactic acids, and polyglyconate acids. If formed of a biodegradable polymer, the degradation should be slow enough so that the nut 10 will remain strong and securely tightened at least for the duration of healing of the bone. If the orthopedic nut 10 will subsequently biodegrade, it is not be necessary for the surgeon to open and gain access on the nut side of the bone, and the bone screw may be longitudinally retracted after the bone heals while leaving the biodegradable nut 10 in place.

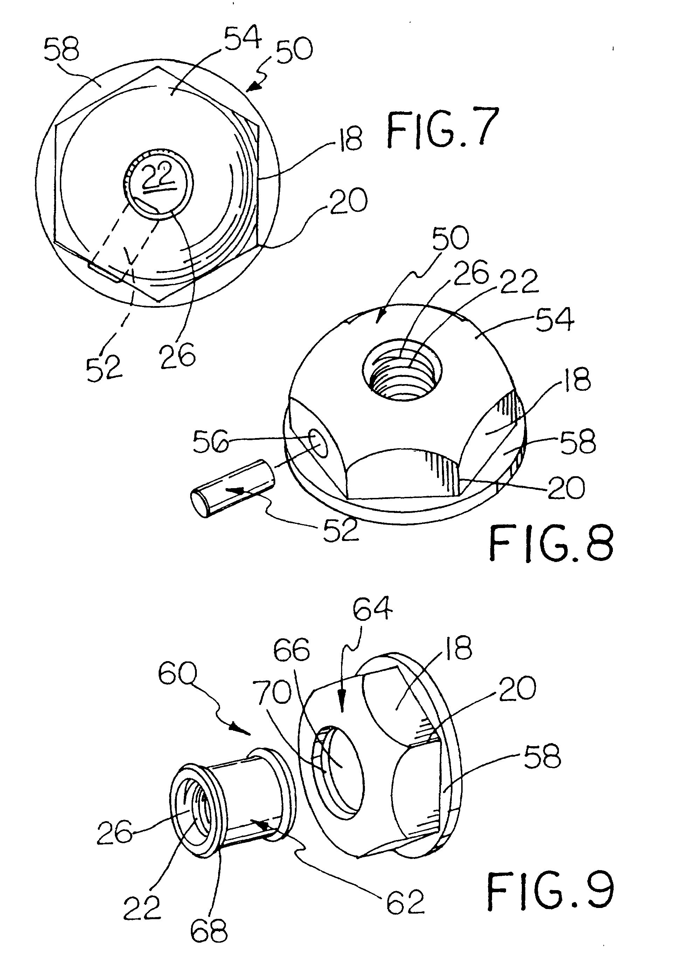

FIGS. 7 and 8 show an alternative head portion 50 in accordance with the present invention. Rather than having the entirety of head portion 50 formed of a polymer, only an interference component such as an insert or pin 52 is formed of the bio-compatible polymer. The remaining nut portion 54 of the head portion 50 may be formed of a bio-compatible metaL The threads 26 in the nut portion 54 may be formed with ample clearance for the intended bone screw. Preferably after forming the nut portion 54 with threads 26 and flats 18, an insert hole 56 is machined into the nut portion in intersection with the screw hole 22. The pin 52 is press fit or otherwise secured into the insert hole 56 such that at least a portion of the pin 52 extends beyond the major diameter of the threads 26.

The head portion 50 may be used in conjunction with the washer 14 of the previous embodiments, or a washer portion 58 may be integrally formed with the nut portion 54. Upon use of the head portion 50, the bone screw contacts and taps threads into the end of the pin 52. Similar to the previously-described embodiments, the compressibility of the pin 52 and the tight fit obtained by having the bone screw cut threads into the pin 52 allow the pin 52 to perform a locking function for the head portion 50.

FIG. 9 shows an alternative head portion 60 in accordance with the present invention. In this embodiment, only a threaded insert 62 is formed of the biocompatible polymer. The remaining nut portion 64 of the head portion 60 may be formed of a bio-compatible metal. The threaded insert 62 snaps into a central hole 66 of the nut portion 64. The threaded insert 62 may be rotationally secured to the nut portion 64 due to a press fit, or with an adhesive. Alternatively, the threaded insert 62 may have a non-cylindrical outer shape (not shown) which positively mates with a correspondingly-shaped central hole in the nut portion 64, to prevent rotation of the threaded insert 62 relative to the nut portion 64. The threaded insert 62 includes a protrusion 68 which bears against a shoulder 70 on the nut portion 64 to transfer the force of the bone screw to the nut portion 64.

The head portion 60 may be used in conjunction with the washer 14 of the previous embodiments, or a washer portion 72 may be integrally formed with the nut portion 64. Upon use of the head portion 60, the bone screw is received in the threaded insert 62. Similar to the previously-described embodiments, the compressibility of the threaded insert 62 and/or and the tight fit obtained by having the bone screw cut threads into the threaded insert 62 allow the threaded insert 62 to perform a locking function for the head portion 60.

Although the present invention has been described with reference to preferred embodiments, workers skilled in the art will recognize that changes may be made in form and detail without departing from the scope of the invention as defined by the accompanying claims.

## Claims

1. An orthopedic nut (10) for a bone screw comprising:
a head portion (12),
the head portion comprising:
a head (16) of a shape adapted to be received in a torqueing tool for rotation about a head axis (24) ; and
a sleeve (28) formed integrally with the head (16) and extending axially about the head axis (24), **characterised in that** the head portion is formed at least in part of a bio-compatible polymer adapted to be cut by a self-tapping thread of a bone screw which is harder than the polymer, and **in that** the orthopedic nut (10) further comprises:
a washer (14) attached to the sleeve (28) of the head portion (12), the washer for bearing against a bone, the washer (14) having an opening defined therein and aligned with the head axis (24) due to the attachment of the washer to the sleeve, the opening for receiving the bone screw therethrough, the washer (14) having a shoulder (30) with a bearing surface larger than the head (16).

2. The orthopedic nut of claim 1, wherein the washer (14) is formed of a radiopaque material.

3. The orthopedic nut of claim 2, wherein the washer (14) is formed of titanium.

4. The orthopedic nut of claim 1, 2, or 3, wherein the washer (14) is formed separately from the head portion (12).

5. The orthopedic nut of any preceding claim, wherein the sleeve (28) comprises a protrusion (34) and wherein the washer (14) comprises a recess (36) mating with the protrusion to attach the washer to the sleeve (28).

6. The orthopedic nut of any preceding claim ,wherein the head portion (12) is rotatable relative to the attached washer (14).

7. The orthopedic nut of any preceding claim, wherein a circular opening (22) is defined in the head portion (12) about the head axis (24), the circular opening having an inner diameter, and further comprising:
screw threads (26) formed on the inner diameter of the circular opening for receiving threads of a bone screw.

8. The orthopedic nut of any preceding claim, wherein the head portion (12) is formed of ultra high molecular weight polyethylene.

9. The orthopedic nut of any preceding claim, wherein the head (16) is shaped hexagonally.

## Patentansprüche

1. Orthopädische Mutter (10) für eine Knochenschraube mit
einem Kopfabschnitt (12), der umfasst:
einen Kopf (16) mit einer Form, die in einem Drehmomentwerkzeug für eine Drehung um eine Kopfachse (24) aufgenommen werden kann; und
eine Hülse (28), die einstückig mit dem Kopf (16) ausgebildet ist und sich axial um die Kopfachse (24) erstreckt, **dadurch gekennzeichnet, dass** der Kopfabschnitt zumindest teilweise aus einem biologisch kompatiblem oder verträglichem Polymer gebildet ist, das durch ein selbstschneidendes Gewinde einer Knochenschraube, die Härter als das Polymer ist, geschnitten werden kann, und dass die orthopädische Mutter (10) eine Scheibe (14) umfasst, die an der Hülse (28) des Kopfabschnitts (12) angebracht ist, um die Scheibe an einem Knochen zu halten oder zu tragen, wobei die Scheibe (14) eine Öffnung aufweist, die darin definiert ist und mit der Kopfachse (24) aufgrund der Anbringung der Scheibe an der Hülse ausgerichtet ist, wobei die Öffnung zum Aufnehmen der Knochenschrauben ausgelegt ist und die Scheibe (14) eine Schulter (30) aufweist, deren Trag- oder Haltefläche größer als der Kopf (16) ist.

2. Orthopädische Mutter nach Anspruch 1, bei der die Scheibe (14) aus einem röntgenstrahlungsundurchlässigem Material gebildet ist.

3. Orthopädische Mutter nach Anspruch 2, bei der die Scheibe (14) aus Titan gebildet ist.

4. Orthopädische Mutter nach Anspruch 1, 2 oder 3, bei der die Scheibe (14) von dem Kopfabschnitt (12) getrennt gebildet ist.

5. Orthopädische Mutter nach einem der vorangegangenen Ansprüche, bei der die Hülse (28) einen Vorsprung (34) aufweist und die Scheibe (14) eine Aussparung (36) umfasst, die zum Anbringen der Scheibe an die Hülse (28) an den Vorsprung angepasst ist.

6. Orthopädische Mutter nach einem der vorangegangenen Ansprüche, bei welcher der Kopfabschnitt relativ zu der angebrachten Scheibe (14) drehbar ist.

7. Orthopädische Mutter nach einem der vorangegangenen Ansprüche, bei der eine kreisförmige Öffnung (22) in dem Kopfabschnitt (12) um die Kopfachse (24) definiert ist, wobei die kreisförmige Öffnung einen Innendurchmesser aufweist und außerdem umfasst:
ein Schraubengewinde (26), das an dem Innendurchmesser der kreisförmigen Öffnung zum Aufnehmen des Gewindes einer Knochenschraube gebildet ist.

8. Orthopädische Mutter nach einem der vorangegangenen Ansprüche, bei welcher der Kopfabschnitt (12) aus einem Polyethylen mit einem extrem hohen Molekulargewicht gebildet ist.

9. Orthopädische Mutter nach einem der vorangegangenen Ansprüche, bei welcher der Kopf (16) hexagonal geformt ist.

## Revendications

1. Ecrou orthopédique (10) pour une vis à os comprenant :
une partie tête (12),
la partie tête comprenant :
une tête (16) d'une forme conçue pour être reçue dans un outil de serrage pour effectuer une rotation autour d'un axe de tête (24) ; et
une chemise (28) formée intégralement avec la tête (16) et s'étendant axialement autour de l'axe de tête (24), **caractérisée en ce que** la partie tête est formée au moins en partie d'un polymère biocompatible qui est plus dur que le polymère conçu pour être coupé par un écrou autotaraudeur d'une vis à os, et **en ce que** l'écrou orthopédique (10) comprend, en outre :
une rondelle (14) fixée à la chemise (28) de la partie tête (12) pour appuyer contre un os, la rondelle (14) ayant une ouverture définie dans celle-ci et alignée avec l'axe de tête (24) de par la fixation de la rondelle à la chemise, l'ouverture étant destinée à recevoir la vis à os à l'intérieur, la rondelle (14) ayant un épaulement (30) ayant un surface de support plus large que la tête (16).

2. Ecrou orthopédique selon la revendication 1, dans lequel la rondelle (14) est formée d'un matériau radio-opaque.

3. Ecrou orthopédique selon la revendication 2, dans lequel la rondelle (14) est formée de titane.

4. Ecrou orthopédique selon la revendication 1, 2 ou 3, dans lequel la rondelle (14) est formée séparément de la partie tête (12).

5. Ecrou orthopédique selon l'une quelconque des revendications précédentes, dans lequel la chemise (28) comprend une protubérance (34) et dans lequel la rondelle (14) comprend un évidement (36) s'accouplant avec la protubérance pour fixer la rondelle à la chemise (28).

6. Ecrou orthopédique selon l'une quelconque des revendications précédentes, dans lequel la partie tête (12) peut entrer en rotation par rapport à la rondelle (14) fixée.

7. Ecrou orthopédique selon l'une quelconque des revendications précédentes, dans lequel une ouverture circulaire (22) est définie dans la partie tête (12) autour de l'axe de tête (24), l'ouverture circulaire ayant un diamètre interne, et comprenant, en outre :
des filetages de vis (26) formés sur le diamètre interne de l'ouverture circulaire pour recevoir les filetages d'une vis à os.

8. Ecrou orthopédique selon l'une quelconque des revendications précédentes, dans lequel la partie tête (12) est formée de polyéthylène à poids moléculaire ultra élevé.

9. Ecrou orthopédique selon l'une quelconque des revendications précédentes, dans lequel la tête (16) est de forme hexagonale.
